# EUROPEAN PATENT APPLICATION

(11) **EP 1 666 470 A1**
(43) Date of publication of application: **07.06.2006**
(21) Application number: 04771819.2
(22) Date of filing: 12.08.2004
(51) Int. Cl.: C07D 251/34

(54) **PROCESS FOR PRODUCTION OF N,N';N''-TRISUBSTITUTED ISOCYANURIC ACIDS**

(30) Priority: 02.09.2003 JP 2003310474; 09.09.2003 JP 2003316546; 02.12.2003 JP 2003402697
(71) Applicant: DAICEL CHEMICAL INDUSTRIES, LTD., Sakai-shi, Osaka 590-8501 (JP)
(72) Inventor: HIRAI, Naruhisa c/o Research Center, Himeji-shi, Hyogo 671-1283 (JP)
(74) Representative: Grünecker, Kinkeldey, Stockmair & Schwanhäusser Anwaltssozietät
(86) International application number: PCT/JP2004/011858
(87) International publication number: WO 2005/023784

(57) **Abstract**

A process produces an N,N',N"-trisubstituted isocyanuric acid represented by following Formula (4): 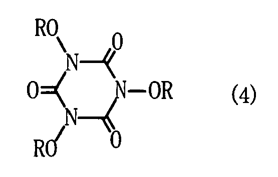 wherein R is a hydroxyl-protecting group, by heating an N-substituted carbamic acid derivative represented by following Formula (1): 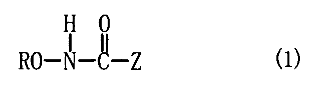 wherein R has the same meaning as defined above; and Z is a group represented by following Formula (2) or (3): 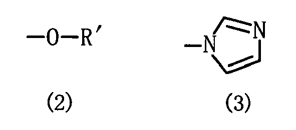 wherein R' is a hydrocarbon group or a heterocyclic group having a carbon atom at the bonding site with the adjacent oxygen atom, wherein the heating is carried out at a temperature in a range of 95°C to 145°C where Z is the group represented by Formula (3). This process can easily and conveniently produce the N,N',N"-trisubstituted isocyanuric acid in a high yield.

## Description

### Technical Field

The present invention relates to processes for producing N,N',N"-trisubstituted isocyanuric acids which are useful typically as adsorbents, catalysts for various reactions or precursors thereof, and intermediates for resins.

### Background Art

N,N',N"-trihydroxyisocyanuric acid has been conventionally used as an adsorbent for sulfur dioxide (e.g., Japanese Unexamined Patent Application Publication No. 04-250819 and PCT Japanese Translation Patent Publication No. 06-502349) and an oxidation catalyst (e.g., PCT International Publication No. WO 03/55600).

As an example of methods for producing an N,N',N"-trialkylisocyanuric acid, a method of heating and thereby trimerizing an isocyanate in the presence typically of a base is known. However, this method as intact cannot be applied to the production of N,N',N"-trihydroxyisocyanuric acid or N,N',N"-trialkoxyisocyanuric acid, since a raw material hydroxyisocyanate or alkoxyisocyanate has not yet been isolated as product.

Certain methods are known as methods for producing an N,N',N"-trialkoxyisocyanuric acid by trimerizing an alkoxyisocyanate derivative having a protected hydroxyl group, as an equivalent to hydoxyisocyanate. Angew. Chem. 1961, 73, 657 discloses that N,N',N"-tris (benzyloxy) isocyanuric acid is produced by reacting carbonyldiimidazole with O-benzylhydroxylamine and heating the reaction mixture to 90°C. PCT International Publication No. WO 03/55600 discloses a similar method, but the yield of N,N',N"-tris (benzyloxy) isocyanuric acid is still as low as 19%. As reactions for use in the production of an N,N',N"-trialkoxyisocyanuric acid, Croat. Chem. Acta 2000, 73, 569 discloses a reaction of using benzotriazole, phosgene and O-benzylhydroxylamine; Canadian. J. Chem. 1960, 38, 343 discloses a method of reacting phosgene with an O-alkylhydroxylamine and treating the reaction product with triethylamine; Tetrahedron Lett. 1968, 40, 4315 discloses a reaction by the action of methyl azide formate and light; and J. Org. Chem. 1965, 30, 1268 discloses a reaction using sodium diethyl N-alkoxyphosphoramidate and carbon dioxide.

### Disclosure of Invention

However, these methods are not always satisfactory, because they require complex raw materials, produce an N,N',N"-trialkoxyisocyanuric acid in an insufficient yield or have poor operability in reaction or aftertreatment.

Accordingly, an object of the present invention is to provide a process for easily and conveniently producing an N,N',N"-trisubstituted isocyanuric acid in a high yield.

Another object of the present invention is to provide a process for producing a high-purity N,N',N"-trisubstituted isocyanuric acid in a high yield.

After intensive investigations to achieve the above objects, the present inventors have found that an N,N',N"-trisubstituted isocyanuric acid can be easily and conveniently produced in a high yield by using an N-substituted carbamic acid ester as a raw material or hating a specific N-substituted carbamic acid derivative at a temperature in a specific range. The present invention has been accomplished based on these findings.

Specifically, the present invention provides a process for producing an N,N',N"-trisubstituted isocyanuric acid, comprising the step of heating an N-substituted carbamic acid derivative represented by following Formula (1): 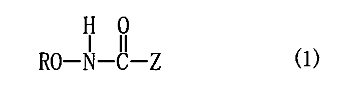 wherein R is a hydroxyl-protecting group; and Z is a group represented by following Formula (2) or (3): 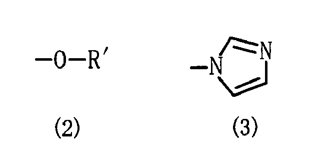 wherein R' is a hydrocarbon group or a heterocyclic group having a carbon atom at the bonding site with the adjacent oxygen atom, wherein the heating step is carried out at a temperature in a range of 95°C to 145°C where Z is the group represented by Formula (3), to thereby form an N,N',N"-trisubstituted isocyanuric acid represented by following Formula (4): 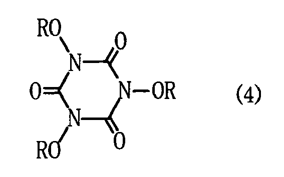 wherein R has the same meaning as defined above.

The present invention further provides, in another aspect, a process for producing an N,N',N"-trisubstituted isocyanuric acid, comprising the step of heating an O-substituted hydroxylamine represented by following Formula (C):

RO-NH₂ (C)

wherein R is a hydroxyl-protecting group, or a salt thereof with (I) a compound represented by following Formula (A) or a compound represented by following Formula (B): 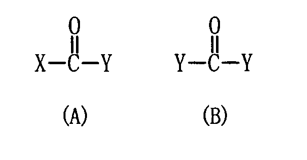 wherein X is a halogen atom; and Y is NH₂ or OR', wherein R' is a hydrocarbon group or a heterocyclic group having a carbon atom at the bonding site with the adjacent oxygen atom, or with (II) dimethyl carbonate, urea or phosgene, and a hydroxy compound represented by following Formula (D):

R'OH (D)

wherein R' has the same meaning as defined above, to thereby form an N,N',N"-trisubstituted isocyanuric acid represented by following Formula (4): 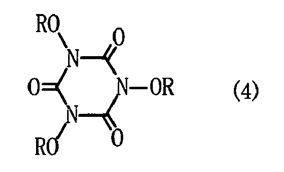 wherein R has the same meaning as defined above.

In a yet another aspect, the present invention provides a process for producing an N,N',N"-trisubstituted isocyanuric acid, comprising the steps of reacting a carbonyldiimidazole represented by following Formula (5): 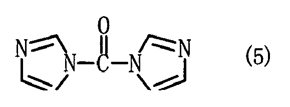 with an O-substituted hydroxylamine represented by following Formula (6):

R-ONH₂ (6)

wherein R is a hydroxyl-protecting group, or a salt thereof, and further heating at a temperature in a range of 95°C to 145°C, to thereby form an N,N',N"-trisubstituted isocyanuric acid represented by following Formula (4): 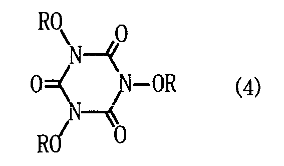 wherein R has the same meaning as defined above.

In each of the production processes, the reaction may be carried out in the presence of a base.

Above-mentioned R is preferably an arylmethyl group which may be substituted, and R' is preferably an aromatic cyclic group which may be substituted.

In addition, the present invention provides a process for producing an N,N',N"-trisubstituted isocyanuric acid, comprising the step of purifying an N,N',N"-trisubstituted isocyanuric acid represented by following Formula (4): 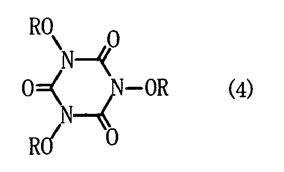 wherein R is a hydroxyl-protecting group by at least one purification means selected from crystallization, repulping and washing, with the use of an alcohol-containing solvent.

The "hydroxyl-protecting group" as used in the present description includes a group which is not intended for leaving or elimination, in addition to a group which will be eliminated to form hydroxyl group.

According to the production processes of the present invention, N,N',N"-trisubstituted isocyanuric acids can be easily and conveniently produced in high yields, andhigh-purity N,N',N"-trisubstituted isocyanuric acids can be produced in high yields. The resulting N,N',N"-trisubstituted isocyanuric acids can be used over a wide range typically as absorbents and catalysts for a variety of reactions.

### Best Mode for Carrying out the Invention

According to the present invention, the N-substituted carbamic acid derivative represented by Formula (1) is used as a raw material. In Formula (1), R is a hydroxyl-protecting group, and Z is the group represented by Formula (2) or (3). In Formula (2), R' is a hydrocarbon group or a heterocyclic group having a carbon atom at the bonding site with the adjacent oxygen atom.

Any hydroxyl-protecting group conventionally used in the field of organic synthesis can be used as the hydroxyl-protecting group in R. Examples of such protecting groups are hydrocarbon groups each having 1 to 30 carbon atoms (preferably 1 to 20 carbon atoms) such as alkyl groups (e.g., methyl, ethyl, propyl, isopropyl, butyl, t-butyl and other alkyl groups each having 1 to 30 carbon atoms (preferably 1 to 20 carbon atoms)), alkenyl groups (e.g., allyl group), cycloalkylgroups (e.g., cyclohexyl group), aryl groups (e.g., 2,4-dinitrophenyl and trimethylphenyl groups), aralkyl groups (e.g., arylmethyl groups which may be substituted); and groups which have 2 to 20 carbon atoms and can form acetal or hemiacetal group with hydroxyl group, such as substituted methyl groups (e.g., methoxymethyl, t-butoxymethyl, methylthiomethyl, benzyloxymethyl, 2-methoxyethoxymethyl, 2,2,2-trichloroethoxymethyl, bis(2-chloroethoxy)methyl, and 2-(trimethylsilyl)ethoxymethyl groups), substituted ethyl groups (e.g., 1-ethoxyethyl, 1-methyl-1-methoxyethyl, 1-isopropoxyethyl, 2,2,2-trichloroethyl, 2-methoxyethyl and 1-[methoxyethoxy]ethyl groups), tetrahydropyranyl group, tetrahydrofuranyl group, and 1-hydroxyalkyl groups (e.g., 1-hydroxyethyl, 1-hydroxyhexyl, 1-hydroxydecyl, 1-hydroxyhexadecyl and 1-hydroxy-1-phenylmethyl groups).

Other examples of the hydroxyl-protecting group are acyl groups (e.g., aliphatic saturated or unsaturated acyl groups including aliphatic acyl groups each having 1 to 20 carbon atoms such as formyl, acetyl, propionyl, butyryl, isobutyryl, valeryl, pivaloyl, hexanoyl, heptanoyl, octanoyl, nonanoyl, decanoyl, lauroyl, myristoyl, palmitoyl, and stearoyl groups; acetoacetyl group; alicyclic acyl groups including cycloalkanecarbonyl groups such as cyclopentanecarbonyl and cyclohexanecarbonyl groups; and aromatic acyl groups such as benzoyl and naphthoyl groups), sulfonyl groups (e.g., methanesulfonyl, ethanesulfonyl, trifluoromethanesulfonyl, benzenesulfonyl, p-toluenesulfonyl and naphthalenesulfonyl groups), alkoxycarbonyl groups (e.g., C₁-C₄ alkoxy-carbonyl groups such as methoxycarbonyl, ethoxycarbonyl and t-butoxycarbonyl groups), aralkyloxycarbonyl groups (e.g., benzyloxycarbonyl and p-methoxybenzyloxycarbonyl groups), substituted or unsubstituted carbamoyl groups (e.g., carbamoyl, methylcarbamoyl and phenylcarbamoyl groups), groups each corresponding to an inorganic acid (e.g., sulfuric acid, nitric acid, phosphoric acid or boric acid) except for removing OH group, dialkylphosphinothioyl groups (e.g., dimethylphosphinothioyl group), diarylphosphinothioyl groups (e.g., diphenylphosphinothioyl group), substituted silyl groups (e.g., tri-(C₁-C₁₀ hydrocarbon group) -substituted silyl groups such as trimethylsilyl, triethylsilyl, t-butyldimethylsilyl, tribenzylsilyl and triphenylsilyl groups), and heterocyclic groups.

Preferred examples of R include hydrocarbon groups; groups that can form acetal or hemiacetal group with hydroxyl group, such as substituted methyl groups, substituted ethyl groups, tetrahydropyranyl group and tetrahydrofuranyl group; acyl groups; aralkyloxycarbonyl groups; and substituted silyl groups. Among them, hydrocarbon groups such as arylmethyl groups which may be substituted are typically preferably used.

Examples of the arylmethyl groups which may be substituted are arylmethyl groups represented by following Formula (a): 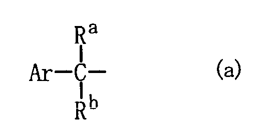 wherein R^{a} and R^{b} are each hydrogen atom or a hydrocarbon group; and Ar is an aryl group.

Examples of the hydrocarbon group in R^{a} and R^{b} are alkyl groups including C₁-C₄ alkyl groups such as methyl, ethyl, isopropyl and t-butyl groups; alkenyl groups including C₂-C₄ alkenyl groups such as vinyl and allyl groups; alkynyl groups; alicyclic hydrocarbon groups; and aromatic hydrocarbon groups such as phenyl and naphthyl groups. Examples of the aryl group in R are aromatic hydrocarbon groups such as phenyl, naphthyl, anthryl and phenanthryl groups; and aromatic heterocyclic groups such as pyridine group.

Each of R^{a}, R^{b} and Ar may be substituted. Examples of such substituents are halogen atoms; hydrocarbon groups such as alkyl groups, aryl groups and cycloalkyl groups; alkoxy groups such as alkoxy groups including C₁-C₄ alkoxy groups such as methoxy group, and aryloxy groups such as phenoxy group; carboxyl group; substituted oxycarbonyl groups such as alkoxycarbonyl groups including C₁-C₄ alkoxy-carbonyl groups such as methoxycarbonyl and ethoxycarbonyl groups; acyl groups; acyloxy groups such as acetyloxy group; nitro group; hydroxyl group; mercapto group; substituted thio groups; substituted or unsubstituted carbamoyl groups; cyano group; substituted or unsubstituted amino groups such as amino group and N,N-di-C₁-C₄ alkylamino groups including N,N-dimethylamino group; sulfo group; heterocyclic groups, and groups comprising these groups combined with each other.

At least two of the substituents of Ar may be combined to form an aromatic or nonaromatic ring together with carbon atom(s) constituting Ar. Examples of such rings are 5- to 12-membered aromatic or nonaromatic rings, particularly hydrocarbon rings (including fused carbon rings and bridged carbon rings) or heterocyclic rings (including fused heterocyclic rings and bridged heterocyclic rings) having about 6 to 10 members. Each of these rings may have one or more substituents. Examples of such substituents are alkyl groups, haloalkyl groups, hydroxyl group, alkoxy groups, carboxyl group, substituted oxycarbonyl groups, acyl groups, acyloxy groups, nitro group, cyano group, amino groups and halogen atoms.

Typical examples of the arylmethyl group which may be substituted are benzyl; halobenzyl such as 2, 6-dichlorobenzyl and 3-bromobenzyl; nitrobenzyl such as 2-nitrobenzyl; diphenylmethyl, and triphenylmethyl groups. Preferably, an arylmethyl group having about 7 to about 20 carbon atoms is used.

Examples of the hydrocarbon group in R' are hydrocarbon groups each having 1 to 30 carbon atoms, of which those having 1 to 20 carbon atoms are preferred. Specific examples thereof are alkyl groups including C₁-C₄ alkyl groups such as methyl, ethyl, isopropyl and t-butyl groups; alkenyl group including C₁-C₄ alkenyl groups such as vinyl and allyl groups; alkynyl groups; alicyclic hydrocarbon groups such as cyclohexyl group; aromatic hydrocarbon groups such as phenyl, naphthyl, anthryl and phenanthryl groups; and groups comprising a plurality of these groups combined with each other, including aralkyl groups such as benzyl and phenethyl groups.

The heterocyclic ring constituting the heterocyclic group having a carbon atom at the bonding site with the adjacent oxygen atom includes an aromatic heterocyclic ring and a nonaromatic heterocyclic ring. Examples of such aromatic heterocyclic rings are heterocyclic rings containing oxygen atom as a hetero atom, including 5-membered rings such as furan, tetrahydrofuran, oxazole and isoxazole, 6-membered rings such as 4-oxo-4H-pyran, tetrahydropyran and morpholine, fused rings such as benzofuran, isobenzofuran, 4-oxo-4H-chromene, chroman and isochroman; heterocyclic rings containing sulfur atom as a hetero atom, including 5-membered rings such as thiophene, thiazole, isothiazole and thiadiazole, 6-membered rings such as 4-oxo-4H-thiopyran, and fused rings such as benzothiophene; heterocyclic rings containing nitrogen atom as a hetero atom, including 5-membered rings such as pyrrole, pyrrolidine, pyrazole, imidazole and triazole, 6-membered rings such as pyridine, pyridazine, pyrimidine, pyrazine, piperidine and piperazine, and fused rings such as indole, indoline, quinoline, acridine, naphthyridine, quinazoline and purine.

Each of these hydrocarbon groups and heterocyclic groups having a carbon atom at the bonding site with the adj acent oxygen atom may be substituted. Examples of such substituents are similar groups to those which R^{a} and other groups in Formula (a) may have. Specific examples thereof are halogen atoms, hydrocarbon groups, alkoxy groups, carboxyl group, substituted oxycarbonyl groups, acyl groups, acyloxy groups, nitro group, hydroxyl group, mercapto group, substituted thio groups, substituted or unsubstituted carbamoyl groups, cyano group, substituted or unsubstituted amino groups, sulfo group, heterocyclic groups, and groups comprising these groups combined with each other. At least two of these substituents may be combined to form a ring together with atom (s) constituting the hydrocarbon group or heterocyclic group. Examples of the ring herein are the aromatic or nonaromatic rings which the substituents of Ar may form.

Preferred examples of R' are aromatic hydrocarbon groups which may be substituted and aromatic heterocyclic groups which may be substituted, namely, aromatic cyclic groups which may be substituted, for easier elimination of -OR' by heating and higher reaction rate. Typical examples of such aromatic cyclic groups which may be substituted are phenyl, chlorophenyl, tolyl, xylyl, methoxyphenyl, nitrophenyl, naphthyl, 2-furanyl and 4-pyridyl groups. Among them, aromatic cyclic groups each having about 6 to about 20 carbon atoms are preferably used.

Examples of reactions for use in the production of N-substituted carbamic acid derivatives represented by Formula (1) in which Z is the group represented by Formula (2) (N-substituted carbamic esters) are: (i) a reaction of the compound represented by Formula (A), such as haloformic acid ester or haloformamide, or the compound represented by Formula (B), such as a carbonic acid ester, urea or carbamic acid ester, with the O-substituted hydroxylamine represented by Formula (C): RO-NH₂ or a salt thereof; (ii) a reaction of dimethyl carbonate, urea or phosgene with the hydroxy compound represented by Formula (D): R'OH and the O-substituted hydroxylamine represented by Formula (C): RO-NH₂ or a salt thereof; and (iii) a reaction of a carbamic acid ester represented by following Formula (E): 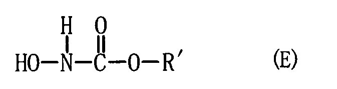 wherein R' has the same meaning as defined above with an alkyl halide represented by Formula (F): RX, wherein X is a halogen atom; and R has the same meaning as defined above, or a compound having a double bond, such as an alkene corresponding to R, in which the carbamic acid ester of Formula (E) is formed by the reaction of the compound represented by Formula (A), such as haloformic acid ester or haloformamide, or the compound represented by Formula (B), such as a carbonic acid ester, urea or carbamic acid ester, with the O-substituted hydroxylamine represented by Formula (C): RO-NH₂ or a salt thereof. For example, an N-benzyloxycarbamic acid ester such as phenyl N-benzyloxycarbamate can be formed from a haloformic acid ester such as phenyl chloroformate with O-benzylhydroxylamine hydrochloride by using the reaction (i).

Where necessary, the reactions (i), (ii) and (iii) may be carried out in the presence of a base or a catalyst such as an transesterification catalyst. Examples of the base herein are bases exemplified later, such as sodium acetate, pyridine and sodium ethoxide. The amount of the base can be suitably set in a range not adversely affecting the reaction and is generally about 0 to about 10 equivalents, preferably about 0 to about 5 equivalents and more preferably about 0 to about 2 equivalents to the O-substituted hydroxylamine or a salt thereof.

The reactions may be carried out in the presence of a solvent. The solvent herein can be any solvent that is inert to the reaction and includes solvents exemplified later.

The reaction temperature is, for example, -20°C to 100°C, preferably 0°C to 60°C, and more preferably 0°C to 40°C. The reaction can be carried out under ordinary pressure, under reduced pressure or under a pressure (under a load) and is generally carried out under ordinary pressure. The reaction can be performed according to any procedure such as batch procedure, semibatch procedure or continuous procedure.

If necessary, the reaction product after the completion of the reaction can be separated and purified typically by separation means such as filtration, concentration, distillation, extraction, crystallization, recrystallization or column chromatography, or separation means comprising these means in combination.

As a result of the reaction (i), (ii) or (iii), a corresponding N-substituted carbamic acid ester is prepared. The N-substituted carbamic acid ester used as a raw material in the present invention can be produced any suitable process not limited to the above production processes.

The N-substituted carbamic acid derivatives represented by Formula (1) in which Z is the group represented by Formula (3) can be produced, for example, by (iv) a reaction of the carbonyldiimidazole represented by Formula (5) with the O-substituted hydroxylamine represented by Formula (6) or a salt thereof. Examples of the salt of the O-substituted hydroxylamine are hydrochloride, phosphate, sulfate, nitrate and oxalate. The amount of the carbonyldiimidazole in the reaction is not specifically limited but is generally about 0.01 to about 10 equivalents, preferably about 0.1 to about 5 equivalents, and more preferably about 0.5 to about 2 equivalents, to the O-substituted hydroxylamine or a salt thereof.

The reaction may be carried out in the coexistence of a base. In particular, a base is preferably used where a salt of the O-substituted hydroxylamine is used as a raw material. Bases mentioned later can be used as the base. Each of these bases can be used alone or in combination.

The amount of the base can be set in a range not adversely affecting the reaction, but is generally about 0 to about 10 equivalents, preferably about 0 to about 5 equivalents, and more preferably about 0 to about 2 equivalents, to the O-substituted hydroxylamine or a salt thereof.

The reaction may be carried out in the presence of a solvent. The solvent herein can be any solvent that is inert to the reaction and includes solvents exemplified later. The amount of the solvent can be suitably set in a range not adversely affecting the reaction, but is generally about 100 to about 10000 parts by weight, preferably about 200 to about 8000 parts by weight, and more preferably about 300 to about 5000 parts by weight, to 100 parts by weight of the O-substituted hydroxylamine or a salt thereof.

The reaction temperature is, for example, -20°C to 100°C, preferably 0°C to 60°C, and more preferably 0°C to 40°C. The reaction can be carried out under ordinary pressure, under reduced pressure or under a pressure (under a load) and is generally carried out under ordinary pressure. The reaction can be performed according to any procedure such as batch procedure, semibatch procedure or continuous procedure.

If necessary, the reaction product after the completion of the reaction can be separated and purified typically by separation means such as filtration, concentration, distillation, extraction, crystallization, recrystallization or column chromatography, or separation means comprising these means in combination.

By the above-mentioned process, a corresponding N-substituted carbamic acid derivative is prepared from the carbonyldiimidazole and the O-substituted hydroxylamine or a salt thereof. The N-substituted carbamic acid derivative [the compound in which Z is the group represented by Formula (3)] used as a raw material in the present invention can be produced by any process not limited to the above production process.

According to the present invention, the N,N',N"-trisubstituted isocyanuric acid represented by Formula (4) is formed by heating the N-substituted carbamic acid derivative represented by Formula (1), wherein heating is carried out at a temperature in a range of 95°C to 145°C where Z is the group represented by Formula (3) [hereinafter referred to as "Production Process 1"]. The N-substituted carbamic acid derivative may be a separated and purified product, a reaction mixture containing the N-substituted carbamic acid derivative or a concentrate thereof. For example, the N,N',N"-trisubstitutedisocyanuric acid represented by Formula (4) can be formed by heating a reaction mixture as a result of the reaction between the carbonyldiimidazole represented by Formula (5) and the O-substituted hydroxylamine represented by Formula (6) or a salt thereof, or a concentrate thereof at a temperature in a range of 95°C to 145°C.

In Production Process 1 according to the present invention, the reaction is preferably carried out in the presence of a base. The addition of the base can increase the reaction rate. Examples of the base are inorganic bases and organic bases, including tertiary amines such as triethylamine, 4-dimethylaminopyridine (DMAP), 1,8-diazabicyclo[5.4.0]-7-undecene (DBU), triethylenediamine (1,4-diazabicyclo[2.2.2]octane; DABCO), 1,5-diazabicyclo[4.3.0]-5-nonene (DBN), hexamethylenetetramine, tetramethylethylenediamine, trioctylamine, dimethylaniline, N-methylpyrrolidine, N-methylpiperidine and 4-methylmorpholine; nitrogen-containing aromatic heterocyclic compounds such as pyridine, lutidine, picoline and imidazole; alkali metal hydroxides such as sodium hydroxide and potassium hydroxide; alkaline earth metal hydroxides such as calcium hydroxide and magnesium hydroxide; oxides of alkali metal or alkaline earth metal, such as magnesium oxide and calcium oxide; carbonates of alkali metal or alkaline earth metal, such as potassium hydrogen carbonate, sodium hydrogen carbonate, potassium carbonate and sodium carbonate; carboxylates of alkali metal or alkaline earth metal, such as sodium acetate and potassium acetate; and alkoxides of alkali metal or alkaline earth metal, such as sodiummethoxide, potassiummethoxide, sodium ethoxide, potassium ethoxide, t-butoxysodium and t-butoxypotassium. Each of these bases can be used alone or in combination. Among them, preferred examples are tertiary amines such as triethylamine and 4-dimethylaminopyridine; and nitrogen-containing aromatic heterocyclic compounds such as lutidine and picoline. The base may be one derived from the substrate (reaction raw material).

The amount of the base can be suitably set in a range not adversely affecting the reaction, but is generally about 0 to 10 equivalents (e.g., about 0.01 to about 10 equivalents), preferably about 0 to about 5 equivalents (e.g., about 0.1 to 5 equivalents), and more preferably about 0 to about 2 equivalents (e.g., about 0.5 to 2 equivalents), to the N-substituted carbamic acid derivative.

The reaction may be carried out in the presence of a solvent. The solvent can be any solvent that is inert to the raw material. Examples thereof are organic solvents including aromatic hydrocarbons which may be substituted, such as benzene, toluene, xylenes, nitrobenzene and chlorobenzene; aliphatic hydrocarbons such as pentane, hexane and heptane; alicyclic hydrocarbons which may be substituted, such as cyclohexane and methylcyclohexane; esters such as ethyl acetate and butyl acetate; ethers such as diethyl ether, dioxane, tetrahydrofuran (THF) and diphenyl ether; halogenated hydrocarbons such as carbon tetrachloride, chloroform and methylene chloride; nitriles such as acetonitrile and benzonitrile; and N,N-dimethylformamide, and mixtures of these organic solvents. The amount of the solvent is suitably set in a range not adversely affecting the reaction, but is generally about 0 to about 10000 parts by weight (e.g., about 0 to about 8000 parts by weight), preferably about 0 to about 5000 parts by weight, more preferably, particularly where the N-substituted carbamic acid derivative is an N-substituted carbamic acid ester, about 0 to about 1000 parts by weight, and typically preferably about 0 to about 300 parts by weight, to 100 parts by weight of the N-substituted carbamic acid derivative.

The reaction temperature is, for example, 20°C to 200°C, preferably 40°C to 160°C, and more preferably 60°C to 140°C. An excessively low reaction temperature may invite a decreased conversion, and an excessively high reaction temperature may invite decomposition of the product, thus inviting a decreased yield in any case. However, where the raw material is the compound of Formula (1) in which Z is the group represented by Formula (3), the reaction temperature should fall in a range of 95°C to 145°C. In this case, a reaction temperature lower than 95°C invite a decreased reaction rate (reaction speed), and one exceeding 145°C invites decomposition of the product N,N',N"-trisubstituted isocyanuric acid, thus inviting a decreased yield. The reaction temperature in this case is preferably about 100°C to about 140°C.

The reaction can be carried out under ordinary pressure, under reduced pressure or under a pressure (under a load) and is generally carried out under ordinary pressure. The reaction can be performed according to any procedure such as batch procedure, semibatch procedure or continuous procedure.

According to the above-mentioned process, the N-substituted carbamic acid derivative represented by Formula (1) is trimerized to form the N,N',N"-trisubstituted isocyanuric acid represented by Formula (4). Where the N-substituted carbamic acid derivative represented by Formula (1) is the N-substituted carbamic acid ester in which Z is the group represented by Formula (2), -OR' group is eliminated as an alcohol or phenol by heating to thereby form a trimer.

The N,N',N"-trialkoxyisocyanuric acid of Formula (4) in which R is a hydroxyl-protecting group can be converted into an N,N',N"-trihydroxyisocyanuric acid of Formula (4) in which R is hydrogen atom by deprotecting the hydroxyl-protecting group in R. For example, N,N',N"-trihydroxyisocyanuric acid can be formed by reacting N,N',N"-tris(benzyloxy)isocyanuric acid with a hydrogenation decomposition catalyst such as a palladium catalyst and hydrogen. N,N',N"-trihydroxyisocyanuric acid can also be prepared by hydrolyzing an N,N',N"-trisubstituted isocyanuric acid in which R is, for example, an alkyl group, substituted methyl group, substituted ethyl group, acyl group or substituted silyl group.

According to the present invention, the N,N',N"-trisubstitutedisocyanuric acid represented by Formula (4) can also be produced by heating the O-substituted hydroxylamine represented by Formula (C): RO-NH₂ or a salt thereof with (I) the compound represented by Formula (A) or the compound represented by Formula (B), or (II) dimethyl carbonate, urea or phosgene, and a hydroxy compound represented by Formula (D): R'OH (hereinafter referred to as "Production Process 2"). Examples of the salt of the O-substituted hydroxylamine are those mentioned above. Examples of the compound represented by Formula (A) are haloformic acid esters and haloformic acid amides (haloformamides). Examples of the compound represented by Formula (B) are carbonic acid esters, urea and carbamic acid esters.

The reaction is preferably carried out in the presence of a base and may be carried out in the presence of a solvent. If necessary, a catalyst such as a transesterification catalyst may be added to the reaction system. The conditions of the reaction, such as type and amount of the base, type and amount of the solvent and temperatures, can be those exemplified in Production Process 1. The reaction can be carried out under ordinary pressure, under reduced pressure or under a pressure (under a load). Where a carbonic acid ester is used as the raw material, the reaction under ordinary pressure or reduced pressure while distilling off a by-produced alcohol or phenol may generally lead to a higher yield. The reaction can be performed according to any procedure such as batch procedure, semibatch procedure or continuous procedure.

According to the above-mentioned process, the N,N',N"-trisubstituted isocyanuric acid represented by Formula (4) is produced from the O-substituted hydroxylamine represented by Formula (C) and the compound represented by Formula (A) or compound represented by Formula (B). The N,N',N"-trisubstituted isocyanuric acid is also produced from the O-substituted hydroxylamine represented by Formula (C), dimethyl carbonate or urea, and the hydroxy compound represented by Formula (D). According to Production Process 2, the target N,N',N"-trisubstituted isocyanuric acid can be produced from, for example, a carbonic acid ester and O-substituted hydroxylamine in one step.

The reaction product after the completion of the reaction can be separated and purified typically by separation means such as filtration, concentration, distillation, extraction, crystallization, recrystallization or column chromatography, or separation means comprising these means in combination.

Another production process according to the present invention includes the step of purifying an N, N', N"-trisubstituted isocyanuric acid represented by Formula (4) by at least one purification means selected from crystallization, repulping and washing, with the use of an alcohol-containing solvent. Each of crystallization, repulping and washing can be used alone or in combination as the separation means.

Examples of a crude N,N',N"-trisubstituted isocyanuric acid to be purified are reaction products, such as a reaction mixture or treated product thereof, formed by heating the N-substituted carbamic acid derivative represented by Formula (1). This purification process is typically useful where R is an arylmethyl group which may be substituted.

The alcohol-containing solvent is preferably a solvent in which unreacted raw materials such as the N-substituted carbamic acid derivative represented by Formula (1) (and a raw material thereof) and R'OH, by-products such as imidazole and R'OH, and the base, if added, are highly soluble, and the product N,N',N"-trisubstituted isocyanuric acid represented by Formula (4) is hardly soluble. For example, solvents containing a lower alcohol having 1 to 6 carbon atoms, typically methanol, are preferred. By using such a solvent, the loss caused by dissolution of the reaction product N,N',N"-trisubstituted isocyanuric acid in the solvent in the purification step can be avoided. The concentration of the alcohol in the alcohol-containing solvent is, for example, 30 percent by weight or more, preferably 50 percent by weight or more, and further preferably 80 percent by weight or more.

Examples of the material to subject to crystallization are a mixture containing the product formed by heating the N-substituted carbamic acid derivative represented by Formula (1), and a mixture containing the product formed by heating the reaction product between the carbonyldiimidazole represented by Formula (5) and the O-substituted hydroxylamine represented by Formula (6) or a salt thereof (hereinafter these mixtures are referred to as "reaction mixture after heating") . The crystallization is carried out typically by dissolving the N,N',N"-trisubstituted isocyanuric acid in the alcohol-containing solvent with heating, and then cooling the solution, or by mixing a solution containing the N,N',N"-trisubstituted isocyanuric acid with the alcohol-containing solvent.

Examples of the material to subject to repulping are the reaction mixture after heating, and N,N',N"-trisubstituted isocyanuric acid which has been recrystallized. An example of the latter is an N,N',N"-trisubstituted isocyanuric acid after crystallization and removing the solvent typically by decantation or filtration. The amount of a solvent for repulping is, for example, about 1 to about 200 times by weight, preferably about 5 to about 100 times by weight, and more preferably about 10 to about 50 times by weight that of the N,N',N"-trisubstituted isocyanuric acid. Repulping is carried out at a temperature of generally about 0°C to 100°C, and preferably about 10°C to about 60°C. Repulping is carried out in a suitable container. The repulping procedure may be carried out more than once. A liquid after repulping (repulped liquid) is removed typically by decantation.

Examples of the material to subject to washing are the reaction mixture after heating, the N,N',N"-trisubstituted isocyanuric acid after crystallization, such as an N,N',N"-trisubstituted isocyanuric acid after recrystallization and removal of the solvent typically by decantation or filtration, and the N,N',N"-trisubstituted isocyanuric acid after the repulping, such as an N,N',N"-trisubstituted isocyanuric acid after repulping and removal of the solvent typically by decantation. The amount of a solvent for washing is, for example, about 1 to about 100 times by weight, and preferably about 2 to about 20 times by weight that of the N,N',N"-trisubstituted isocyanuric acid. Washing is carried out at a temperature of generally about 0°C to about 100°C, and preferably about 10°C to about 60°C. Washing is carried out in a suitable container. The washing procedure may be performed more than once. A liquid after washing (washing) is removed typically by decantation or filtration.

The resulting N,N',N"-trisubstituted isocyanuric acid prepared by crystallization, repulpingorwashingmaybe further subjected to, for example, washing which may be followed by drying.

As a result of the purification step, the unreacted raw materials and by-products such as imidazole attached to the reaction product, and added base and other components can be efficiently removed to thereby yield a high-purity N,N',N"-trisubstituted isocyanuric acid. The purified N,N',N"-trisubstituted isocyanuric acid has a purity of, for example, 95% or more.

### Examples

The present invention will be illustrated in further detail with reference to several examples below, which are not intended to limit the scope of the invention.

### PRODUCTION EXAMPLE 1

### Production of phenyl N-benzyloxycarbamate

In a flask were placed 3.19 g (20 mmol) of O-benzylhydroxylamine hydrochloride, 3.16 g (40 mmol) of pyridine and 40 ml of acetonitrile, followed by stirring at 0°C in an atmosphere of nitrogen. A total of 3.13 g (20 mmol) of phenyl chloroformate was added dropwise from a dropping funnel while holding the temperature of reaction mixture to 0°C to 2°C, followed by stirring at 25°C for 2 hours. The resulting reaction mixture was concentrated, mixed with 50 ml of ethyl acetate, and the precipitate was removed by filtration. After washing the precipitate with 30 ml of ethyl acetate, the filtrate was sequentially washed with 40 ml of a 0.5 N aqueous solution of hydrochloric acid, 20 ml of water and 20 ml of a saturated aqueous solution of sodium chloride. The organic layer was dried over magnesium sulfate and concentrated, followed by removal of the solvent using a vacuum pump. The residue was mixed with 20 ml of hexane, pulverized, filtrated and washed with 10 ml of hexane, followed by suction drying for 1 hour. The subsequent heating and drying at 80°C under reduced pressure for 12 hours yielded 4.56 g (yield: 94%) of phenyl N-benzyloxycarbamate as slightly yellow crystals.

### [Spectral data of phenyl N-benzyloxycarbamate]

¹H-NMR (CDCl₃, 500 MHz) d: 4.96 (s, 2H, CH₂), 7.10-7.16 (m, 2H, ArH), 7.20-7.26 (m, 1H, ArH), 7.32-7.46 (m, 7H, ArH), 7.63 (brs, 1H, NH)
¹³C-NMR (DMSO-d6, 125MHz) d: 155.3, 150.3, 135.1, 129.4, 129.3, 128.8, 128.6, 125.8, 121.3, 78.8

### EXAMPLE 1

A total of 1.60 g (10 mmol) of O-benzylhydroxylamine hydrochloride was mixed with 1.58 g (20 mmol) of pyridine and 20 ml of acetonitrile, followed by stirring at 0°C in an atmosphere of nitrogen. After adding dropwise 1. 57 g (10 mmol) of phenyl chloroformate while holding the temperature of reaction mixture to 0°C to 2°C, the mixture was stirred at 25°C for 2 hours. The reaction mixture was concentrated, mixed with ethyl acetate, the precipitate was filtrated, and the filtrate containing phenyl N-benzyloxycarbamate was concentrated. A total of 1.22 g (10 mmol) of 4-dimethylaminopyridine (DMAP) was added to the concentrated residue, followed by heating on a bath at 120°C for 20 minutes. After cooling, 10 ml of methanol was added, followed by stirring for 30 minutes. The precipitate was filtrated, washed with 10 ml of methanol, and dried under suction for 1 hour. The subsequent heating and drying at 80°C under reduced pressure for 12 hours yielded 1.22 g (yield: 82%) of N,N',N"-tris(benzyloxy) isocyanuric acid as white crystals.

### [Spectral data of N,N',N"-tris(benzyloxy)isocyanuric acid]

¹H-NMR (DMSO-d6, 500 MHz) d: 5.11 (s, 6H, CH₂), 7.4-7.5 (m, 9H, ArH), 7.5-7.6 (m, 6H, ArH)
¹³C-NMR (DMSO-d6, 125MHz) d: 145.0, 133.6, 129.7, 129.2, 128.5, 78.6

### EXAMPLE 2

In a 500-ml four-neck flask equipped with a condenser tube, thermometer and dropping funnel were placed 16.0 g (100 mmol) of O-benzylhydroxylamine hydrochloride, 15.8 g (20 mmol) of pyridine and 200 ml of acetonitrile, followed by stirring at 0°C in an atmosphere of nitrogen. After adding dropwise 15.7 g (100 mmol) of phenyl chloroformate from the dropping funnel while holding the temperature of reaction mixture to 0°C to 2°C, the ice bath was removed, followed by stirring for 2 hours. The reaction mixture was concentrated, mixed with 500 ml of ethyl acetate, and the precipitate was filtrated. After washing the precipitate with 300 ml of ethyl acetate, the filtrate containing phenyl N-benzyloxycarbamate was concentrated. The concentrated residue was mixed with 12.2 g (100 mmol) of 4-dimethylaminopyridine (DMAP), followed by stirring on a bath at 90°C. At the time when the reaction temperature attained the highest (96°C), the temperature of the bath was raised to 120°C, and the mixture was heated for a total of 20 minutes. After cooling, the reaction mixture was mixed with 100 ml of methanol, followed by stirring for 20 minutes. The precipitate was filtrated and washed with 100 ml of methanol, followed by suction drying for 1 hour. The subsequent heating and drying at 80°C under reduced pressure for 12 hours yielded 11.2 g (yield: 75%) of N,N',N"-tris(benzyloxy)isocyanuric acid as white crystals.

### EXAMPLE 3

A total of 0.61 g (5 mmol) of 4-dimethylaminopyridine (DMAP) was added to 1.22 g (5 mmol) of phenyl N-benzyloxycarbamate prepared in EXAMPLE 1, followed by heating on a bath at 120°C for 20 minutes. After cooling, 6 ml of methanol was added, followed by stirring for 20 minutes. The precipitate was filtrated and washed with 6 ml of methanol, followed by suction drying for 1 hour. The subsequent heating and drying at 80°C under reduced pressure for 12 hours yielded 0. 61 g (yield: 81%) of N,N',N"-tris(benzyloxy)isocyanuric acid as white crystals.

### EXAMPLE 4

The procedure of Example 1 was repeated, except for using 10 mmol of triethylamine (TEA) as the base instead of DMAP, to yield 1.04 g (yield: 70%) of N,N',N"-tris(benzyloxy)isocyanuric acid as white crystals.

### EXAMPLE 5

The procedure of Example 1 was repeated, except for using 10 mmol of triethylenediamine (TEDA) as the base instead of DMAP, to yield 0.67 g (yield: 45%) of N,N',N"-tris(benzyloxy)isocyanuric acid as white crystals.

### EXAMPLE 6

The procedure of Example 1 was repeated, except for using 10 mmol of imidazole as the base instead of DMAP, to yield 0.63 g (yield: 42%) of N,N',N"-tris (benzyloxy) isocyanuric acid as white crystals.

### EXAMPLE 7

The procedure of Example 1 was repeated, except for using 10 mmol of lutidine as the base instead of DMAP, to yield 0.46 g (yield: 31%) of N,N',N"-tris (benzyloxy) isocyanuric acid as white crystals.

### EXAMPLE 8

The procedure of Example 1 was repeated, except for using 10 mmol of 1,8-diazabicyclo[5.4.0]undecene (DBU) as the base instead of DMAP and heating at 90°C, instead of 120°C, for 30 minutes, to yield 0.42 g (yield: 28%) of N,N',N"-tris(benzyloxy)isocyanuric acid as white crystals.

### EXAMPLE 9

The procedure of Example 1 was repeated, except for using 15 mmol of DMAP as the base, to yield 0.94 g (yield: 63%) of N,N',N"-tris(benzyloxy)isocyanuric acid as white crystals.

### EXAMPLE 10

The procedure of Example 1 was repeated, except for using 5 mmol of DMAP as the base, to yield 0.65 g (yield: 44%) of N,N',N"-tris(benzyloxy)isocyanuric acid as white crystals.

### EXAMPLE 11

The procedure of Example 1 was repeated, except for heating at 140°C, instead of 120°C, for 10 minutes, to yield 0.99 g (yield: 66%) of N,N',N"-tris(benzyloxy)isocyanuric acid as white crystals.

### EXAMPLE 12

The procedure of Example 1 was repeated, except for heating at 90°C, instead of 120°C, for 30 minutes, to yield 0. 99 g (yield: 66%) of N,N',N"-tris(benzyloxy)isocyanuric acid as white crystals.

### EXAMPLE 13

The procedure of Example 1 was repeated, except for heating at 160°C, instead of 120°C, to yield 0.34 g (yield: 23%) of N,N',N"-tris(benzyloxy)isocyanuric acid as white crystals.

### EXAMPLE 14

A total of 2.14 g (10 mmol) of diphenyl carbonate was mixed with 1.21 g (10 mmol) of O-benzylhydroxylamine and 1.22 g (10 mmol) of 4-dimethylaminopyridine (DMAP), followed by heating onabathat 120°C for 20minutes. After cooling, 10 ml of methanol was added, followed by stirring for 30 minutes. The precipitate was filtrated and washed with 10 ml of methanol, followed by suction drying for 1 hour. The subsequent heating and drying at 80°C under reduced pressure for 12 hours yielded 0. 39 g (yield: 26%) of N,N',N"-tris(benzyloxy)isocyanuric acid as white crystals.

### EXAMPLE 15

The procedure of Example 14 was repeated, except that the reaction was carried out at a reduced pressure of 100 mmHg, to yield 0.69 g (yield: 46%) of N,N',N"-tris(benzyloxy)isocyanuric acid as white crystals.

### EXAMPLE 16

A total of 21.4 g (100 mmol) of diphenyl carbonate was mixed with 16.0 g (100 mmol) of O-benzylhydroxylamine hydrochloride and 24.4 g (200 mmol) of 4-dimethylaminopyridine (DMAP), followed by heating on a bath at 120°C for 30 minutes. After cooling, 100 ml of methanol was added, followed by stirring for 30 minutes. The precipitate was filtrated and washed with 100 ml of methanol, followed by suction drying for 1 hour. The subsequent heating and drying at 80°C under reduced pressure for 12 hours yielded 10.2 g (yield: 69%) of N,N',N"-tris(benzyloxy)isocyanuric acid as white crystals.

### EXAMPLE 17

A total of 2.14 g (10 mmol) of diphenyl carbonate was mixed with 1.60 g (10 mmol) of O-benzylhydroxylamine hydrochloride and 2.02 g (20 mmol) of triethylamine, followed by heating on a bath at 100°C for 20 minutes. After cooling, 10 ml of methanol was added, followed by stirring for 30 minutes. The precipitate was filtrated and washed with 10 ml of methanol, followed by suction drying for 1 hour. The subsequent heating and drying at 80°C under reduced pressure for 12 hours yielded 0. 77 g (yield: 52%) of N,N',N"-tris(benzyloxy)isocyanuric acid as white crystals.

### EXAMPLE 18

After purging a 500-ml four-neck flask equipped with a condenser tube, thermometer, septum and rotor with nitrogen, 23.9 g (150 mmol) of O-benzylhydroxylamine hydrochloride, 26.8 g (165mmol) of carbonyldiimidazole and 250 ml of tetrahydrofuran (THF) were placed, followed by stirring at 10°C in an atmosphere of nitrogen for 6 hours, to thereby yield 1-(N-benzyloxycarbamoyl)imidazole. The precipitated crystals were filtrated, the filtrate containing 1-(N-benzyloxycarbamoyl)imidazole was concentrated, and the resulting concentrate was reacted at 120°C for 20 minutes. After cooling, 100 ml of methanol was added thereto, followed by stirring for 0.5 hour. The crystals were filtrated and washed with 100 ml of methanol, followed by suction drying for 1 hour. The subsequent heating and drying at 80°C under reduced pressure for 12 hours yielded 12.3 g (yield: 55%) of N, N' , N"-tris (benzyloxy) isocyanuric acid having a purity of 98% as white crystals.

### [Spectral data of N,N',N"-tris(benzyloxy)isocyanuric acid]

¹H-NMR (DMSO-d6, 500 MHz) d: 5.11 (s, 6H, CH₂), 7.4-7.5 (m, 9H, ArH), 7.5-7.6 (m, 6H, ArH)
¹³C-NMR (DMSO-d6, 125MHz) d: 145.0, 133.6, 129.7, 129.2, 128.5, 78.5
MS (FAB⁺) m/z 448 ((M+H)⁺, 21), 371 (16), 181 (22), 129 (12), 91 (100), 57 (11)

### COMPARATIVE EXAMPLE 1

The procedure of Example 18 was repeated, except that the reaction (heating) of the concentrate was carried out at 150°C instead of 120°C, to yield 1.0 g (yield: 4.5%) of N,N',N"-tris(benzyloxy)isocyanuric acid having a purity of 96% as white crystals.

## Claims

1. A process for producing an N,N',N"-trisubstituted isocyanuric acid, comprising the step of heating an N-substituted carbamic acid derivative represented by following Formula (1): 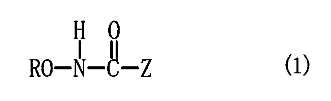 wherein R is a hydroxyl-protecting group; and Z is a group represented by following Formula (2) or (3): 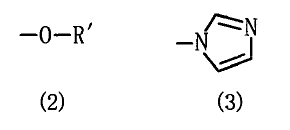 wherein R' is a hydrocarbon group or a heterocyclic group having a carbon atom at the bonding site with the adjacent oxygen atom, wherein the heating step is carried out at a temperature in a range of 95°C to 145°C where Z is the group represented by Formula (3), to thereby form an N,N',N"-trisubstituted isocyanuric acid represented by following Formula (4): 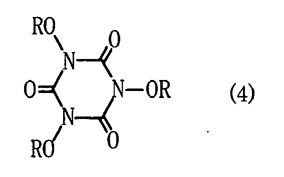 wherein R has the same meaning as defined above.

2. A process for producing an N,N',N"-trisubstituted isocyanuric acid, comprising the step of heating an O-substituted hydroxylamine represented by following Formula (C) :
RO-NH₂ (C)
wherein R is a hydroxyl-protecting group, or a salt thereof with (I) a compound represented by following Formula (A) or a compound represented by following Formula (B): 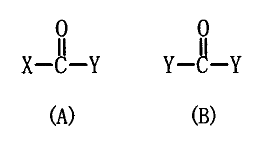 wherein X is a halogen atom; and Y is NH₂ or OR' wherein R' is a hydrocarbon group or a heterocyclic group having a carbon atom at the bonding site with the adjacent oxygen atom, or with (II) dimethyl carbonate, urea or phosgene, and a hydroxy compound represented by following Formula (D):
R'OH (D)
wherein R' has the same meaning as defined above to thereby form an N,N',N"-trisubstituted isocyanuric acid represented by following Formula (4): 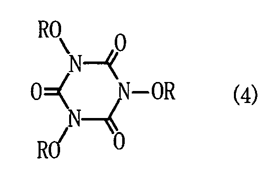 wherein R has the same meaning as defined above.

3. A process for producing an N,N',N"-trisubstituted isocyanuric acid, comprising the steps of reacting a carbonyldiimidazole represented by following Formula (5): 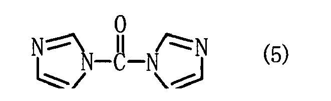 with an O-substituted hydroxylamine represented by following Formula (6):
R-ONH₂ (6)
wherein R is a hydroxyl-protecting group, or a salt thereof, and further heating at a temperature in a range of 95°C to 145°C, to thereby form an N,N',N"-trisubstituted isocyanuric acid represented by following Formula (4): 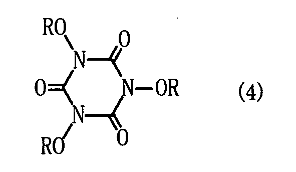 wherein R has the same meaning as defined above.

4. The process for producing an N,N',N"-trisubstituted isocyanuric acid according to any one of claims 1 to 3, wherein the reaction is carried out in the presence of a base.

5. The process for producing an N,N',N"-trisubstituted isocyanuric acid according to any one of claims 1 to 4, wherein R is an arylmethyl group which may be substituted.

6. The process for producing an N,N',N"-trisubstituted isocyanuric acid according to claim 1 or 2, wherein R' is an aromatic cyclic group which may be substituted.

7. A process for producing an N,N',N"-trisubstituted isocyanuric acid, comprising the step of purifying an N,N',N"-trisubstituted isocyanuric acid represented by following Formula (4): 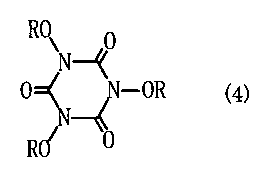 wherein R is a hydroxyl-protecting group by at least one purification means selected from crystallization, repulping and washing, with the use of an alcohol-containing solvent.
